(19) Europäisches Patentamt
European Patent Office
Office européen des brevets

(11) **EP 2 187 851 B1**

(12) **EUROPÄISCHE PATENTSCHRIFT**

(45) Veröffentlichungstag und Bekanntmachung des Hinweises auf die Patenterteilung:
**22.06.2016 Patentblatt 2016/25**

(21) Anmeldenummer: **08801972.4**

(22) Anmeldetag: **10.09.2008**

(51) Int Cl.:
***A61F 9/007*** *(2006.01)*

(86) Internationale Anmeldenummer:
**PCT/EP2008/007400**

(87) Internationale Veröffentlichungsnummer:
**WO 2009/036917 (26.03.2009 Gazette 2009/13)**

(54) **PHAKOEMULSIFIKATIONSVORRICHTUNG**

PHACOEMULSIFICATION DEVICE

DISPOSITIF DE PHACOÉMULSION

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **13.09.2007 DE 102007043612**

(43) Veröffentlichungstag der Anmeldung:
**26.05.2010 Patentblatt 2010/21**

(73) Patentinhaber: **Carl Zeiss Meditec AG**
**07745 Jena (DE)**

(72) Erfinder:
 • **HEYMANN, Manfred**
 **89250 Heidenheim (DE)**
 • **EICHLER, Michael**
 **73434 Aalen (DE)**
 • **DÜVER, Erik**
 **73447 Oberkochen (DE)**
 • **KRAUS, Martin**
 **73460 Hüttlingen (DE)**

(74) Vertreter: **Braunger, Dieter**
**Carl Zeiss AG**
**Patentabteilung**
**Carl-Zeiss-Straße 22**
**73447 Oberkochen (DE)**

(56) Entgegenhaltungen:
**EP-A- 0 394 583     US-A- 5 331 951**
**US-A- 5 394 047     US-A- 5 728 130**
**US-A- 6 027 515     US-B1- 6 203 516**

**Beschreibung**

Die Erfindung betrifft eine Phakoemulsifikationsvorrichtung zum Emulsifizieren einer Linse, ein Verfahren zum Betreiben der Phakoemulsifikationsvorrichtung und ein Phakoemulsifikationssystem mit einer solchen Phakoemulsifikationsvorrichtung.

Die operative Behandlung eines Kataraktes wird heute vorwiegend durch Phakoemulsifikation durchgeführt. Dabei wird eine eingetrübte Augenlinse mittels einer Hohlnadel, welche im Ultraschallbereich schwingt, derart in kleine Bestandteile zertrümmert (emulsifiziert), dass diese Bestandteile durch die Nadel abgesaugt werden können. Anschließend setzt der Operateur als Ersatz für die derart zertrümmerte Linse eine künstliche Linse ein. Eine wesentliche Baugruppe bei der Durchführung einer solchen Phakoemulsifikation ist ein Handstück, welches die genannte Hohlnadel oder auch Schneidspitze aufweist. Die erforderlichen Ultraschallschwingungen zur Zertrümmerung der getrübten Augenlinse können derart erzeugt werden, dass das Handstück mit piezokeramischen Elementen versehen wird. Legt man an diese piezokeramische Elemente eine Spannung an, kann auf Grund des piezoelektrischen Effektes eine Längenänderung bewirkt werden, so dass eine mit dem Piezokeramiken verbundene Nadel in Longitudinalrichtung ausgelenkt werden kann.

Um möglichst große Ampituden der Schneidspitze zu erreichen, werden die piezoelektrischen Elemente im Bereich der Resonanzfrequenz des Handstückes betrieben. Die Resonanzfrequenz eines Handstückes mit einer Schneidspitze kann im lastfreien Zustand sehr genau bestimmt werden. Sobald die Schneidspitze jedoch in Kontakt mit der zu emulsifizierenden Linse tritt, ändern sich die Massenverhältnisse, so dass sich die Resonanzfrequenz verschiebt. Um jeweils möglichst im Bereich der Resonanzfrequenz ein solches Handstück zu betreiben, wird in US 6,997,935 B2 vorgeschlagen, die Phasenlage zwischen der angelegten Spannung und dem fließenden Strom zum Betreiben der piezoelektrischen Elemente zu erfassen und so zu regeln, dass gemäß der Gleichung $P = U * I * \cos \varphi$ eine möglichst maximale Leistung erzielt wird. Damit der Faktor $\cos \varphi$ einen möglichst hohen Betrag einnimmt, muss $\cos \varphi = 1$ bzw. $\varphi = 0$ betragen. Eine solche Situation ist bei einem Resonanzfall gegeben. Verschiebt sich jedoch die Resonanzfrequenz z.B. auf Grund einer Änderung der mechanischen Belastung der Schneidspitze, ist der Phasenwinkel $\varphi \neq 0$, sondern liegt in dem Bereich zwischen 0 und $-\pi/2$ bzw. 0 und + n /2. Gemäß US 6,997,935 B2 wird nach dem Erfassen des Phasenwinkels $\varphi$ die Anregungsfrequenz so geregelt, dass die Anregungsfrequenz der Schneidspitze mit der Eigenfrequenz $\omega_0$ übereinstimmt.

Ein ähnliches System wird in US6203516 vorgeschlagen.

Eine Ursache für die Verlagerung der Resonanzfrequenz ist nicht nur eine veränderte Belastung durch Linsenbruchstücke (Massenänderung), sondern auch eine Erwärmung des Handstückes bei längerem Betrieb sowie eine Alterung der piezokeramischen Elemente und damit Veränderung ihrer physikalischen Eigenschaften. Diese Parameter können sich in beliebiger Form überlagern, so dass permanent nachgeregelt werden muss. Vorteilhaft an einem solchen Verfahren sind der relativ einfache Aufbau eines zugehörigen Verstärkers (Digitalverstärker) und die unkomplizierte Bestimmung des Phasenwinkels. Nachteilig jedoch ist die relativ langsame Regelung, da zur Bestimmung des Phasenwinkels stets mehrere aufeinander folgende Messpunkte des Spannungs- und Stromverlaufs über die Zeit erfasst werden müssen. Außerdem ist der Resonanzfall prinzipiell nie exakt erreichbar. Die Schneidspitze wird stets mit einer erzwungenen Schwingung betrieben, welche nie genau der Eigenfrequenz des Handstückes entspricht. Die Frequenzabweichung mag zwar gering sein, jedoch ist die damit einhergehende Blindleistung relativ hoch, da die mechanische Leistung kubisch proportional zur Frequenz ist. Die zugeführte elektrische Energie wird somit nicht optimal in eine mechanische Energie und damit in die maximale Amplitude der Schneidspitze transformiert. Zudem sind die Störgrößen wie Massenänderung, Erwärmung, Alterung und Produktionstoleranzen unabhängig voneinander, so dass auch bei einem noch so sorgfältig hergestellten Handstück prinzipiell nie sichergestellt werden kann, dass beim Betreiben des Handstückes die Betriebsfrequenz gleich der Eigenfrequenz ist. Allgemein bedeutet dies einen nicht optimalen Betrieb des Handstückes, welches sich in einem relativ hohen Anteil an Blindleistung darstellt.

Es ist daher die Aufgabe der Erfindung, eine Phakoemulsifikationsvorrichtung und ein Verfahren zum Betreiben einer solchen Vorrichtung sowie ein Phakoemulsifikationssystem mit einer solchen Vorrichtung vorzuschlagen, wobei während des Emulsifikationsprozesses ein möglichst niedriger Anteil an Blindleistung auftritt, so dass ein höherer Wirkungsgrad als bei bisher bekannten Phakoemulsifikationsvorrichtungen erzielt wird.

Die Aufgabe wird durch eine Vorrichtung gemäß dem unabhängigen Anspruch 1, und einem System gemäß Anspruch 10 gelöst. Vorteilhafte Ausführungsformen sind in den Unteransprüchen beschrieben.

Die erfindungsgemäße Phakoemulsifikationsvorrichtung weist auf: Ein Handstück, welches eine Schneidspitze zur Emulsifikation einer Linse aufweist, wobei das Handstück mit piezoelektrischen Elementen zur Auslenkung der Schneidspitze versehen ist, einen Regler, dessen Eingang mit dem Handstück elektrisch verbunden ist, um einen Istwert zu empfangen, der proportional zu der an den piezoelektrischen Elementen induzierten Spannung des stets in der Eigenfrequenz schwingenden Handstückes ist, wobei der Regler geeignet ist, den Istwert mit einem vorgegebenen Sollwert zu vergleichen und somit eine Stellgröße zu bestimmen, einen Leistungsverstärker, dessen Eingang mit dem Regler verbunden ist, um die Stellgröße zu empfangen, und dessen Ausgang mit dem Handstück verbunden ist, wobei gleichzeitig mit dem Empfangen des Istwertes das Handstück ansteuerbar ist und eine Leistungsabgabe an das Handstück erfolgen kann, so dass eine unmittelbare Rückkopplung erreichbar ist und somit das Handstück stets in seiner Eigenfrequenz mit einer der Stellgröße entsprechenden Auslenkung der Schneidspitze schwingen kann.

Indem die induzierte Spannung des in der Eigenfrequenz schwingenden Handstückes abgegriffen und verstärkt wird, erhöht sich die Amplitude der in der Eigenfrequenz schwingenden Schneidspitze. Eine solche Vorrichtung schwingt somit selbständig in der Eigenfrequenz, auch wenn es durch äußerer Einflüsse, wie einer Massenänderung der Linsenbruchstücke, oder durch innere Einflüsse, wie einer Erwärmung der Piezokeramiken, beeinflusst wird. Die Störgrößen führen lediglich dazu, dass sich die Eigenfrequenz verschiebt, wobei das System trotzdem stets in der Eigenfrequenz weiter schwingt. Von Bedeutung ist hierbei, dass ein Istwert erfasst wird und gleichzeitig das Handstück ansteuerbar ist. Es gibt somit keinen Erfassungsmodus, in dem das Handstück abgeschaltet ist. Dem Handstück wird während des Erfassens eines Istwertes ständig und ohne Unterbrechung eine Sollgröße zugeführt und somit eine Leistungsabgabe an das Handstück erzielt. Damit lässt sich eine unmittelbare Rückkopplung erreichen, bei der eine Ausgangsgröße des Handstückes, also der Istwert, direkt dem Eingang des Reglers zuführbar ist. Das Handstück wird im eingeschalteten Zustand immer angesteuert, wobei in diesem Zustand immer ein Istwert der an den piezoelektrischen Elementen induzierten Spannung erfasst wird. Eine solche Regelung ist sehr schnell, da kein Phasenwinkel mehr erfasst werden muss. Die Blindleistung lässt sich deutlich reduzieren, so dass ein höherer Wirkungsgrad erreicht wird.

Vorzugsweise ist zwischen dem Regler und dem Handstück ein Übertrager angeordnet, welcher an seinem Eingang mit dem Handstück und an seinem Ausgang mit dem Regler verbunden ist. Der Übertrager ermöglicht eine qualitativ hohe Signalübertragung, wobei gleichzeitig die Spannung an seinem Ausgang deutlich niedriger sein kann als an seinem Eingang. Da die Piezokeramiken oft im Hochvoltbereich (bis 1000 V) betrieben werden, kann durch Einsatz eines derartigen Übertragers eine galvanisch getrennte Transformation im Niedervoltbereich erzielt werden.

Die Phakoemulsifikationsvorrichtung kann auch derart ausgebildet sein, dass sich die piezoelektrischen Elemente durch einen elektrischen Impuls so anregen lassen, dass eine größere Schwingungsamplitude der Schneidspitze im Vergleich zum weißen Rauschen erreicht wird. Die Grundlage für diese Weiterbildung liegt darin, dass das Handstück und die Schneidspitze auch ohne Energiezufuhr in ihrer Eigenfrequenz schwingen, wobei die Amplitude jedoch sehr klein ist. Das Handstück schwingt dabei im sogenannten "weißen Rauschen" in seiner Eigenfrequenz. Zu Beginn eines Regelungsvorganges kann durch die zusätzlichen elektrischen Impulse die Amplitude der Schneidspitze so erhöht werden, dass die Induktionsspannung einen genügend hohen Betrag erreicht, um vom Regler gut verarbeitet werden zu können.

Vorzugsweise kann die vom Verstärker zugeführte Energie wiederholt vollständig unterbrochen werden, so dass jeweils nach einer bestimmten Zeit eine Ruhephase folgt. Da sich während der Emulsifikation die Linse, wie auch andere mit der Schneidspitze in Kontakt tretende Teile wie zum Beispiel die Hornhaut, auf Grund der Ultraschallschwingungen erwärmen, kann bei einer zu hohen Temperatur eine Schädigung des Auges auftreten. Unter Umständen kann es zu einer Hornhautverbrennung (cornea burn) kommen. Die bei einer solchen Ausführungsform der erfindungsgemäßen Phakoemulsifikationsvorrichtung mit höchster Effizienz zugeführte Energie wird während der Ruhephasen nicht zugeführt, so dass das Handstück, die Schneidspitze und die diese umgebenden Teile durch Konvektion und/oder Wärmeaustausch mit dem Aspirations- oder Irrigationsfluid abkühlen können. Während des gesamten Emulsifikationsprozesses wird somit eine geringe thermische Belastung für das Auge erreicht, wobei trotzdem mit hohem Wirkungsgrad emulsifiziert wird. Es ist vorteilhaft, wenn die Vorrichtung so betreibbar ist, dass je Sekunde eine Anzahl von Ruhephasen erzielbar ist, welche mindestens 1 ist und höchstens der Betrag von 1 Prozent der Eigenfrequenz des Handstückes ist. Bei einer Eigenfrequenz von 40 kHz sind dies somit maximal 400 Ruhephasen je Sekunde.

Ferner kann es vorteilhaft sein, wenn die dem Handstück zugeführte Energie von einer Schwingphase zur darauffolgenden Schwingphase in ihrer Höhe variierbar ist. Damit kann dem Umstand Rechnung getragen werden, dass es unterschiedlich harte, eingetrübte Linsen gibt, die eine unterschiedliche minimale Energiezufuhr erfordern.

Gemäß einer anderen Ausführungsform der Erfindung, kann die vom Verstärker zugeführte Energie so geregelt werden, dass nach einer ersten Schwingphase, in der die Schneidspitze mit einer Amplitude betrieben wird, mit der die Linse emulsifiziert werden kann, eine zweite Schwingphase folgt, in der die Schneidspitze mit einer Amplitude betrieben wird, mit der die Linse nicht emulsifiziert werden kann. Während der zweiten Schwingphase wird somit die Energiezufuhr nicht vollständig unterbrochen. Damit kann nach Ablauf der zweiten Schwingphase die Einschwingzeit der darauf folgenden Schwingphase für die Emulsifikation verkürzt werden.

Vorzugsweise weist das Handstück mit der Schneidspitze eine Eigenfrequenz von 20 bis 100 kHz, besonders bevorzugt 40 kHz, auf und ist so betreibbar, dass die Schneidspitze bei der Emulsifikation eine mechanische Leistung zwischen 0 und 3,4 Watt abgibt.

Die Aufgabe wird auch durch ein Verfahren zum Betreiben der oben genannten Phakoemulsifikationsvorrichtung gelöst, wobei vom Verstärker zugeführte Energie wiederholt vollständig unterbrochen wird, so dass jeweils nach einer Schwingphase eine Ruhephase folgt. Dies bewirkt eine Verringerung der thermischen Belastung für die Teile, welche durch die Ultraschallschwingungen erwärmt werden. Es ist geschickt, wenn die Vorrichtung so betrieben wird, dass je Sekunde eine Anzahl von Ruhephasen vorliegt, welche mindestens 1 ist und höchstens der Betrag von 1 Prozent der Eigenfrequenz des Handstückes ist.

Gemäß einer weiteren Ausführungsform des erfindungsgemäßen Verfahrens wird die vom Verstärker zugeführte Energie so geregelt, dass nach einer ersten Schwingphase, in der die Schneidspitze mit einer Amplitude betrieben mit, mit der die Linse emulsifiziert werden kann, eine zweite Schwingphase folgt, in der die Schneidspitze mit einer Amplitude betrieben wird, mit der die Linse nicht emulsifiziert werden kann. Damit ist eine geringere Zeit erforderlich, um die Amplitude der Schneidspitze von einem maximalen Wert auf einen für die Emulsifikation unbedeutenden Wert abzusenken. Ebenso ist eine geringere Zeit erforderlich, um von diesem Wert mit niedriger Amplitude wieder schnell auf einen maximalen Wert der Amplitude zu erhöhen.

Vorzugsweise wird gemäß einer Weiterbildung des erfindungsgemäßen Verfahrens die dem Handstück zugeführte Energie von einer Schwingphase zu der darauf folgenden Schwingphase in ihrer Höhe variiert. Gemäß einer weiteren Ausführungsform wird die für die zweite Schwingphase erforderliche Energie aus einer Restenergie gebildet, welche aus der während der ersten Schwingphase zugeführten Energie stammt.

Ferner ist es vorteilhaft, wenn die im weißen Rauschen befindlichen oder nur mit geringer Amplitude schwingenden Piezoelemente durch einen elektrischen Impuls so angeregt werden, dass eine größere Schwingungsamplitude der Schneidspitze im Vergleich zum weißen Rauschen bzw. dem Schwingen mit niedriger Amplitude erreicht wird. Damit lässt sich eine bessere Signalverarbeitung erreichen.

Die Aufgabe wird auch durch ein Phakoemulsifikationssystem mit einer Phakoemulsifikationsvorrichtung wie vorstehend beschrieben, einer Irrigationsvorrichtung, einer Aspirationsvorrichtung und einer Steuerungsvorrichtung zum Betreiben der Phakoemulsifikationsvorrichtung, der Irrigationsvorrichtung und der Aspirationsvorrichtung gelöst.

Weitere Vorteile und Ausführungsbeispiele der Erfindung werden nachfolgend anhand schematischer Zeichnungen näher erläutert, in welchen zeigen:

Figur 1    eine schematische Darstellung eines Phakoemulsifikationssystems nach dem Stand der Technik;

Figur 2    eine schematische Darstellung einer Phakoemulsifikationsvorrichtung gemäß der Erfindung;

Figur 3    ein Diagramm mit einem Verlauf der maximalen und minimalen Amplitude der Schneidspitze in Abhängigkeit von ihrer Frequenz;

Figur 4    ein Diagramm zur Illustration eines Einschwing- und Ausschwingverhaltens bei der erfindungsgemäßen Phakoemulsifikationsvorrichtung;

Figur 5    ein Diagramm, welches einen Schwingvorgang beim Betrieb der erfindungsgemäßen Phakoemulsifikationsvorrichtung darstellt;

Figur 6    ein Diagramm bezüglich einer Ausführungsform des erfindungsgemäßen Verfahrens zum Betreiben der Phakoemulsifikationsvorrichtung; und

Figur 7    ein Diagramm bezüglich einer weiteren Ausführungsform des erfindungsgemäßen Verfahrens zum Betreiben der Phakoemulsifikationsvorrichtung.

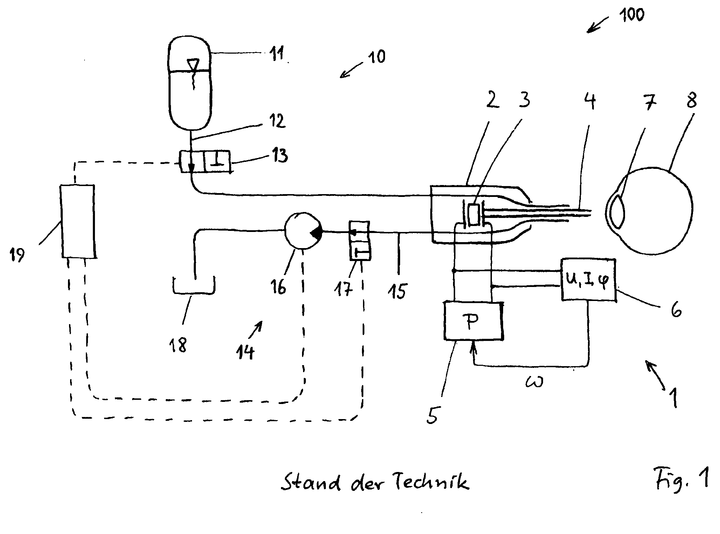

In Figur 1 ist ein System 100 zur Phakoemulsifikation schematisch dargestellt. Das System weist eine Phakoemulsifikationsvorrichtung 1 auf, welche ein Handstück 2, darin enthaltene piezokeramische Elemente 3 und eine daran gekoppelte Schneidspitze 4 aufweist. Die piezokeramischen Elemente 3 sind mit einer Leistungsversorgung 5 verbunden, welche geeignet ist, eine longitudinale Ausdehnung der piezokeramischen Elemente 3 zu bewirken. Der Spannungs- und Stromverlauf in Abhängigkeit von der Zeit und der zugehörige Phasenwinkel $\varphi$ wird mit der Steuerung 6 erfasst, wobei die Anregungsfrequenz $\omega$ so geändert wird, dass der Phasenwinkel $\varphi$ einen möglichst niedrigen Betrag erhält. Dieses Signal mit der veränderten Anregungsfrequenz $\omega$ wird der Leistungsversorgung 5 zugeführt, welche die piezokeramischen Elemente 3 entsprechend betreibt. Die Phakoemulsifikationsvorrichtung 1 ist mit einem Irrigationssystem 10 und einem Aspirationssystem 14 verbunden. Das Irritationssystem 10 weist einen Irrigationsbehälter 11 auf, von dem eine Irrigationsleitung 12 abgeht, deren Zulauf durch ein Irrigationsventil 13 gesteuert werden kann. Das Irrigationsfluid gelangt über die Schneidspitze 4 zu einem Auge 8 mit der zu behandelnden Linse 7. Wenn die Schneidspitze 4 durch Ultraschallschwingungen die Linse 7 emulsifiziert, werden das Irrigationsfluid und die emulsifizierten Partikel durch das Aspirationssystem abtransportiert. Dies erfolgt über eine Leitung 15, welche an eine Aspirationspumpe 16 angeschlossen ist, wobei der Durchfluss durch die Leitung 15 mittels eines Aspirationsventils 17 getrennt werden kann. Das abgesaugte Fluid und die emulsifizierten Partikel werden anschließen einem Behälter 18 zugeführt. Die Ventile 13 und 17 und die Pumpe 16 werden mittels einer Steuerungsvorrichtung betrieben.

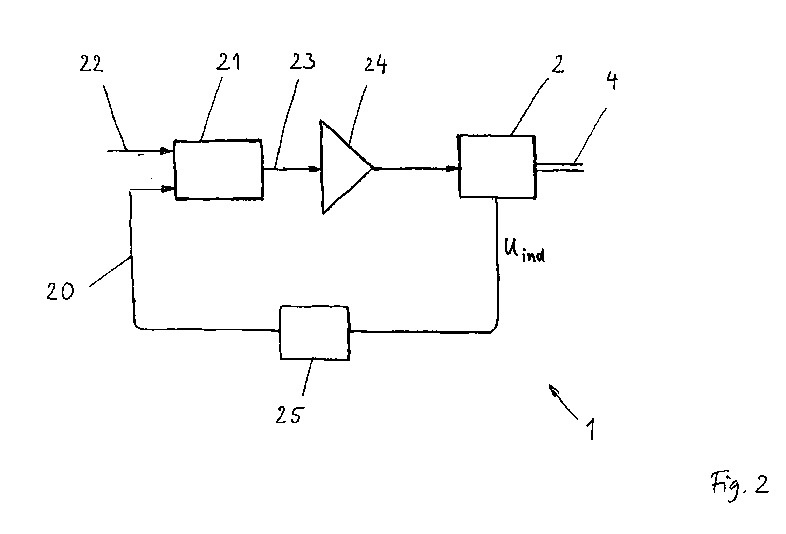

Bei der erfindungsgemäßen Phakoemulsifikationsvorrichtung 1 wird keine Phasenverschiebung $\varphi$ zwischen

einem Spannungs- und Stromverlauf erfasst. Stattdessen wird ein Istwert erfasst, der proportional zu der in den piezoelektrischen Elementen induzierten Spannung des in der Eigenfrequenz schwingenden Handstückes 2 ist, wobei, siehe Figur 2, dieser Istwert über eine Leitung 20 einen Regler 21 zugeführt wird. Der Regler 21 ist geeignet, einen vorgegebenen Sollwert 22, zum Beispiel eine Spannung, welche eine Schwingungsamplitude repräsentiert, die von einem Anwender mit einem Fußpedal vorgegeben wird, mit dem Istwert zu vergleichen und daraufhin eine Stellgröße 23 zu bestimmen, welche einem Leistungsverstärker 24 zugeführt wird. Der Leistungsverstärker 24 ist an seinem Ausgang mit dem Handstück verbunden, so dass das Handstück in seiner Eigenfrequenz mit einer verstärken Auslenkung der Schneidspitze schwingen kann. Durch diese verstärkte Schwingung wird wiederum eine Spannung induziert, welche durch unmittelbare Rückkopplung in gleicher Weise wie vorstehend beschrieben verstärkt wird. Durch einen solchen geschlossenen Regelkreis erhöht sich nach wenigen Durchläufen die Amplitude der Schneidspitze, wobei diese stets in ihrer eigenen Frequenz schwingt.

Nach kurzer Zeit kann die Amplitude so groß werden, dass eine sogenannte Resonanzkatastrophe eintritt, so dass die Piezokeramiken die Amplituden nicht mehr übertragen können und beschädigt werden. Um dies zu vermeiden, muss eine geeignete Leistungsregelung vorgesehen sein. Bei einer drohenden Resonanzkatastrophe kann daher der Verstärkungsfaktor des Leistungsverstärkers auf kleiner als 1 gesetzt werden. Die erfindungsgemäße Phakoemulsifikationsvorrichtung kann dabei so betrieben werden, dass bei sich ändernden Bedingungen zum Beispiel durch Erwärmung, Alterung der Komponenten, und Masseänderung auf Grund der jeweiligen Linsenbruchstücke die Amplitude der Schneidspitze stets konstant ist. Die Amplitude der Schneidspitze ist jeweils direkt proportional zur induzierten Spannung, welche als Regelgröße zum Einsatz kommt.

Wie in "IEC NWIP Requirements for lens removal and vitrectomy devices for ophthalmic surgery", 201.12.4.101.7 Hazardous output for ultrasonic average velocity of TIP" beschrieben ist, ist die maximale zulässige Geschwindigkeit der Schneidspitze auf 20 m/s begrenzt. Somit kann dem Regler 21 eine der Amplitude proportionale induzierte Spannung als Soll-Grenzwert vorgegeben werden.

Im Ruhezustand schwingt das Handstück 2 mit seiner Schneidspitze 4 in seiner Eigenfrequenz, wobei die Amplitude sehr klein ist. Diesen Zustand bezeichnet man als das sogenannte "weiße Rauschen". Sollte die auf Grund dieser sehr kleinen Amplitude erzeugte induzierte Spannung zu niedrig für eine Weiterverarbeitung mit dem Regler 21 sein, kann das Handstück durch einen äußerem Impuls zum Schwingen angeregt werden, so dass eine höhere Amplitude und Induktionsspannung erzielt wird. Die Induktionsspannungen können jedoch auch relativ hoch sein, so dass diese für die Weiterverarbeitung mit dem Regler 21 nicht mehr geeignet sind. Daher kann auch ein Übertrager 25 vorgesehen sein, welcher eine Transformation der Spannungen bewirkt, wobei eine hohe Signalübertragungsrate sichergestellt ist.

Bei der Umsetzung von elektrischer in mechanische Energie im Handstück entsteht Wärme. Eine solche Wärme kann von der Schneidspitze an die Hornhaut und an die eingetrübte Linse übertragen werden und zum Beispiel eine Gefahr für die Hornhaut darstellen (Hornhautverbrennung). Es ist daher vorteilhaft, wenn bei höchstmöglicher Effizienz der Emulsifikation die zusätzlich entstehende Wärme auf ein Minimum reduziert werden kann. Dies lässt sich zum einen durch die Optimierung des Wirkungsgrades erreichen, wie dies bei der erfindungsgemäßen Phakoemulsifikationsvorrichtung möglich ist. Eine weitere Maßnahme kann darin bestehen, die zugeführte Energie in ihrer Gesamtheit bewusst niedrig zu halten. Um trotzdem eine Emulsifikation der Linse zu erreichen, muss eine Mindestenergie zugeführt werden. Ist die zugeführte Energie unterhalb eines Schwellwertes, wird lediglich Wärme in das Auge eingebracht, wobei eine Emulsifiation nicht stattfindet. Oberhalb einer solchen Schwelle findet eine Emulsifikation statt, wobei es dann von der Zahl der Schwingperioden abhängt, bis eine Zertrümmerung der Linse erreicht wird. Die erfindungsgemäße Phako-mulsifikationsvorrichtung kann daher derart betrieben werden, dass nach genügend hohen Amplituden zur Emulsifikation der Linse die Schneidspitze so angesteuert wird, dass die Energiezufuhr vollständig unterbrochen wird. Nach einer Schwingphase folgt somit eine Ruhezeit, in der jedoch unverändert eine Schwingung gemäß dem weißen Rauschen auftritt.

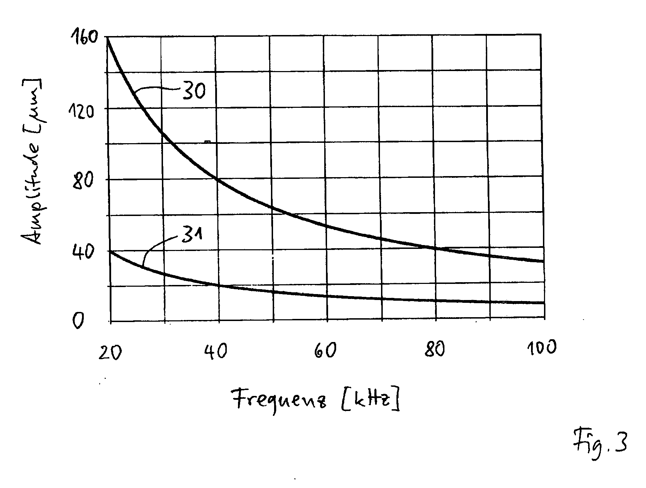

In Figur 3 ist für eine Ausführungsform der Erfindung die jeweils zulässige Amplitude der Schneidspitze in Abhängigkeit von der Zeit dargestellt. Bei dieser Darstellung ist berücksichtigt, dass die maximale Geschwindigkeit der Schneidspitze bei 20 m/s liegt. Wird zum Beispiel die Schneidspitze mit einer Frequenz von 40 kHz betrieben, beträgt die maximale zulässige Amplitude 80 μm, siehe Kurve 30. Die minimale Amplitude bei einer Frequenz von 40 kHz beträgt etwa 20 μm, siehe Kurve 31, wobei ein Betrieb mit einer noch kleineren Amplitude lediglich zu einer geringen Erwärmung, aber nicht mehr zu einer Emulsifikation der Linse führt. Wird die Schneidspitze mit einer maximalen Amplitude $s_{max}$ betrieben, berechnet sich die maximale, longitudinale Spannung $\sigma_{max}$ in der zu emulsifizierenden Linse gemäß

$$\sigma_{max} = \rho\ \omega^2\ s_{max}{}^2 . \qquad (1)$$

Dabei wird die Dichte ρ des Linsenwerkstoffes mit 1000 kg/m$^3$ angenommen, wobei ω die Kreisfrequenz und $s_{max}$ die maximale Amplitude der Schneidspitze ist. Bei einer Frequenz $f = \omega/2\pi$ = 40 kHz und einer maximalen Amplitude

von $s_{max}$ = 80 µm beträgt die maximale longitudinale Spannung $\sigma_{max}$ etwa 0,4 MPa. In Abhängigkeit von der Härte bzw. der Bruchfestigkeit der Augenlinse kann eine geeignete Amplitude der Schneidspitze gewählt werden. Wird bei der gewählten Amplitude die Bruchfestigkeit der Augenlinse erreicht bzw. überschritten, reicht eine einzige Auslenkung der Schneidspitze für eine Zertrümmerung eines Linsenfragmentes aus. Die Härte der Augenlinse kann jedoch stark variieren und auch so hart sein, dass eine Zertrümmerung erst bei wiederholter Auslenkung der Schneidspitze erreicht wird. Die minimale Auslenkung zur Emulsifikation berechnet sich gemäß

$$s_{min} = (\varepsilon \, E / \rho \, \omega^2)^{1/2}, \tag{2}$$

wobei $s_{min}$ die minimale Auslenkung, $\varepsilon$ die Dehnung des Linsenwerkstoffes und E der Elastizitätsmodul des Linsenwerkstoffes bedeuten. Ferner steht $\rho$ für die Dichte des Linsenwerkstoffes und $\omega$ für die Kreisfrequenz. Ist $\varepsilon$ = 0,3 , E = 0,084 N/mm$^2$, $\rho$ = 1000 kg/m$^3$ und $\omega$ = 2 $\pi f$ mit $f$ = 40 kHz, ergibt sich die minimale Auslenkung zu etwa 20 µm, so dass das Verhältnis aus minimaler zu maximaler Auslenkung etwa 25 % beträgt.

Die mechanische Leistung der Schneidspitze, welche auf die Linse übertragen wird, lässt sich gemäß folgender Gleichung berechnen:

$$P = \rho \, \pi^4 \, (D^2 - d^2) f^3 \, s^3 \tag{3}$$

Dabei stehen $\rho$ für die Dichte des Linsenwerkstoffes, D für den Außendurchmesser der rohrförmigen Schneidspitze, $d$ für die Innendurchmesser der Schneidspitze, $f$ für die Eigenfrequenz des Handstückes mit Schneidspitze, und $s$ für die Amplitude der Schneidspitze. Werden als Zahlenwerte die Dichte $\rho$ = 1000 kg/m$^3$, D = 1,2 mm, $d$ = 0,6 mm, $f$ = 40 kHz und $s$ = 80 µm verwendet, ergibt sich eine maximale mechanische Leistung von $P_{max}$ = 3,4 W. Wird nur eine minimale Amplitude von ca. 20 µm (siehe Figur 3) vorgesehen, beträgt die mechanische Leistung $P_{min}$ nur noch 0,054 W, also etwa 1,6 % des maximalen Betrages. Wird weniger als 0,054 W zugeführt, findet keine Emulsifikation mehr statt, sondern nur noch eine relativ geringe Erwärmung der Linse. Wird eine höhere mechanische Leistung als 3,4 W zugeführt, wird der vorgeschriebene Grenzwert einer maximalen Geschwindigkeit von 20 m/s der Schneidspitze überschritten.

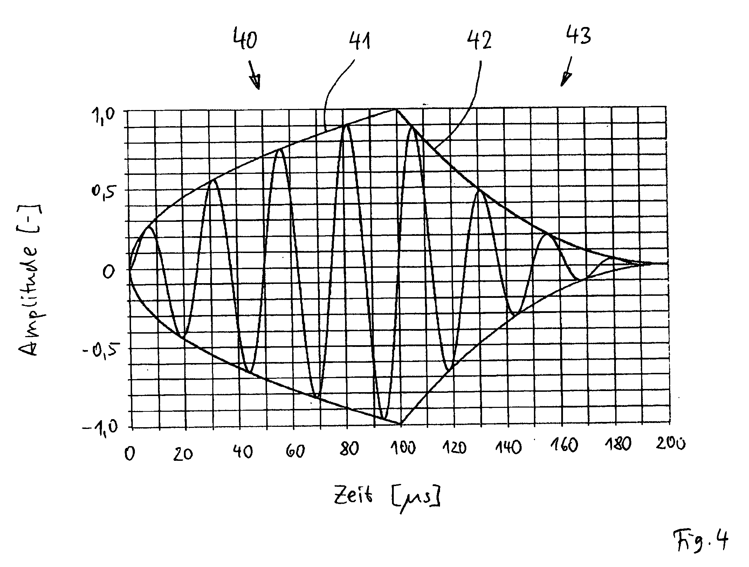

Für den Operateur ist es von Bedeutung, die Augenlinse in möglichst kurzer Zeit mit einer geringen Wärmebelastung zu emulsifizieren. Daher ist es vorteilhaft, den Betrieb der Schneidspitze mit möglichst großer mechanischer Amplitude durchzuführen, um anschließend Ruhephasen zum Abkühlen der erwähnten Komponenten vorzusehen. Den Vorgang des Einschwingens bis zum Erreichen einer maximalen Amplitude und des Abklingens bis zum Erreichen einer Auslenkung auf den Wert 0 zeigt das Diagramm in Figur 4. Die Schneidspitze schwingt jeweils in einer Eigenfrequenz, wobei die Amplituden während des Einschwingvorgangs 40 ständig zunehmen (siehe den Verlauf der Einhüllenden 41). Nach Erreichen der maximalen Amplitude kann durch Unterbrechung der Energiezufuhr die Höhe der Amplituden rasch abnehmen, siehe Einhüllende 42. Bei dem in Figur 4 dargestellten Beispiel nimmt bei dem Ausschwingvorgang 43 nach etwa 4 Schwingperioden die Amplitude auf den Betrag 0 ab. Die erforderliche Zeit für den Einschwing- und Ausschwingvorgang hängt von der Frequenz der Schneidspitze ab. Bei hoher Frequenz wird die maximale Amplitude schneller erreicht als bei niedriger Frequenz.

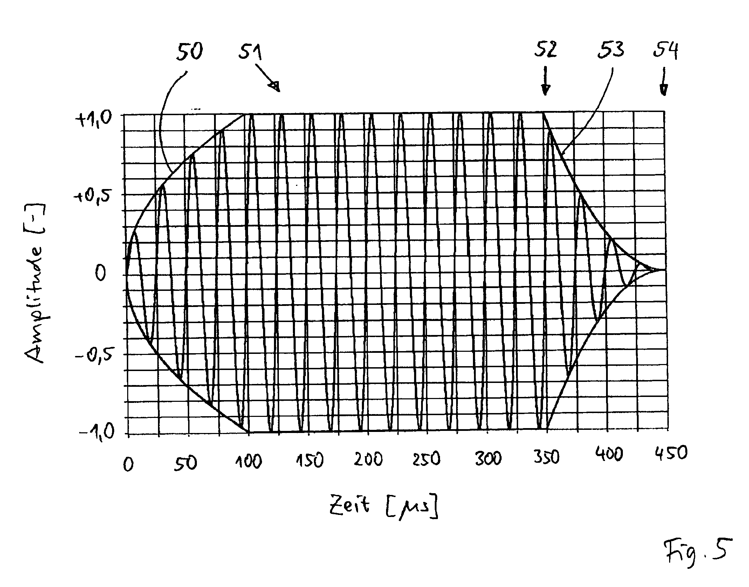

Für eine optimale Emulsifikation kann sowohl die Amplitude der Schwingung als auch die Schwingdauer verändert werden. Bei dem in Figur 5 gezeigten Beispiel wird nach vier Schwingperioden die maximale Amplitude erreicht, siehe Einhüllende 50. Anschließend folgen zehn Schwingperioden, siehe Bezugszeichen 51, in denen die Schneidspitze auf die Linse einwirken kann. Nach Ablauf dieser zehn Schwingperioden, siehe Bezugszeichen 52, wird die Energiezufuhr unterbrochen, so dass die Amplitude der Schneidspitze rasch abnimmt, siehe Einhüllende 53. Nach etwa vier weiteren Schwingperioden ist die Amplitude der Schneidspitze auf den Betrag 0 angelangt, siehe Bezugszeichen 54. Von Bedeutung ist hierbei, dass die Schwingung von ihrem Anfang bis zum Ende jeweils in einer Eigenfrequenz des Handstücks mit Schneidspitze erfolgt.

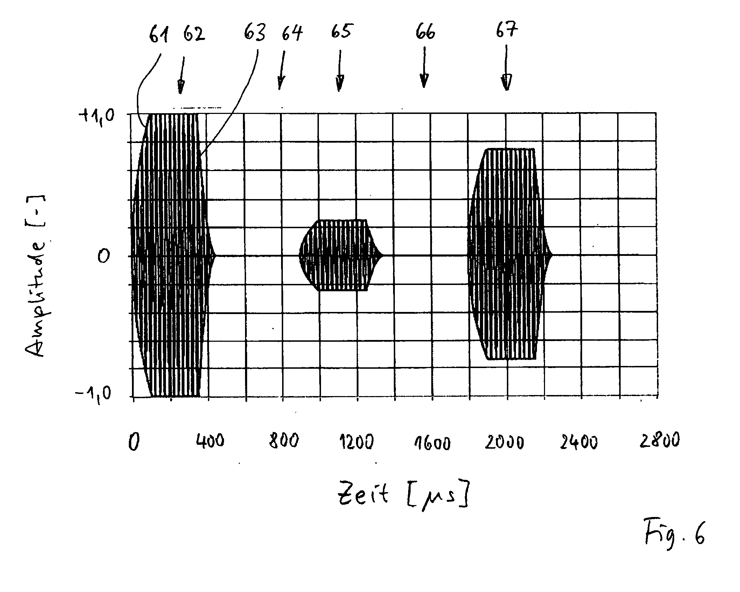

Wenn die Amplitude der Schneidspitze den Betrag 0 erreicht hat, kann für eine gewisse Zeitdauer die Energiezufuhr zu den piezokeramischen Elementen unterbrochen bleiben. Nach Ablauf einer derartigen Ruhephase kann wieder eine Schwingphase folgen, siehe Figur 6. Während einer ersten Schwingphase nimmt die Amplitude der Schneidspitze bis auf ihren maximalen Betrag zu, siehe Einhüllende 61, wobei sie anschließend für eine Zeitdauer von mehreren Schwingperioden auf dieser maximale Amplitude verbleibt, siehe Bezugszeichen 62. Daraufhin wird die Energiezufuhr unterbrochen, so dass die Amplitude der Schneidspitze auf 0 abnimmt, siehe Einhüllende 63. Daraufhin folgt eine Ruhephase 64, auf welche eine zweite Schwingphase folgt, siehe Bezugszeichen 65. Die Amplitude in dieser zweiten Schwingphase 65 kann dabei niedriger als bei der ersten Schwingphase 62 sein. Daraufhin kann erneut eine Ruhephase 66 folgen, an welche sich wieder eine nächste Schwingphase 67 anschließt. Während dieser Schwingphase 67 kann eine Amplitude gewählt werden, welche sich von der Amplitude während der ersten Schwingphase 62 und/oder der zweiten Schwingphase 65 unterscheidet.

Um ein schnelleres Ansteigen bis zum Erreichen der maximalen Amplitude zu erzielen, kann in einer anderen Ausführungsform des Verfahrens zum Betreiben der Phakoemulsifikationsvorrichtung auch während einer Ruhephase 64 oder 66 die Phakoemulsifikationsvorrichtung mit einer niedrigen Schwingamplitude betrieben werden, so dass das Handstück nicht vollkommen in Ruhe ist. Die zugeführte Leistung ist in diesem Zeitraum relativ gering, und die Erwärmung ist vernachlässigbar. Zum Beispiel kann eine Restenergie durch Energierückführung genutzt werden. Der Vorteil dieses Verfahrens liegt darin, dass die Schneidspitze von einer Schwingung mit niedriger Amplitude schneller auf eine Schwingung mit höherer Amplitude geregelt werden kann. Erneut wird darauf hingewiesen, dass während der Schwingphasen das Handstück mit der Schneidspitze jeweils in seiner Eigenfrequenz schwingt.

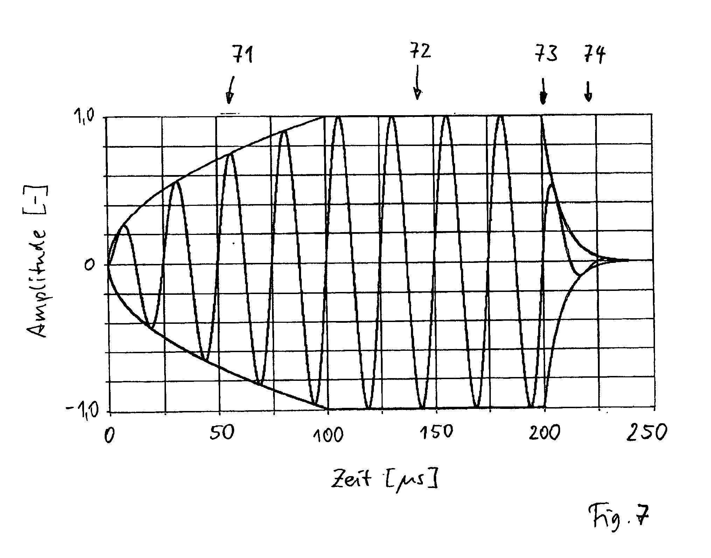

Gemäß einer weiteren Ausführungsform des Verfahrens zum Betreiben der Phakoemulsifikationsvorrichtung wird durch eine Kurzschlussschaltung die Ausschwingzeit reduziert, siehe Figur 7. Nach einem Einschwingen, siehe Bezugszeichen 71, schwingt die Schneidspitze zum Beispiel mit 4 Perioden weiter, siehe Bezugszeichen 72. Nach Abschalten der Energiezufuhr, siehe Bezugszeichen 73, und gezielter Abfuhr der noch vorhandenen Energie ist die Zeit 74 zum Ausschwingen der Schneidspitze kürzer als bei einem normalen Ausschwingvorgang, wie er zum Beispiel in Figur 5 dargestellt ist.

Wenn während der Phakoemulsifikation eine Okklusion oder ein Okklusionsdurchbruch auftritt, ändert sich für kurze Zeit die Eigenfrequenz. Eine solche Frequenzverlagerung kann dazu genutzt werden, um ein derartiges Ereignis zu detektieren. Mit der erfindungsgemäßen Phakoemulsifikationsvorrichtung ist es somit möglich, sehr schnell einen derartigen Vorgang zu detektieren, so dass weitere Subsysteme wie zum Beispiel ein Fluidsystem sehr schnell angesteuert werden können. Damit wird unter anderem eine hohe Stabilität im Auge erzielt.

**Patentansprüche**

1. Phakoemulsifikationsvorrichtung (1), aufweisend:

   - ein Handstück (2), welches eine Schneidspitze (4) zur Emulsifikation einer Linse (7) aufweist, wobei das Handstück (2) mit piezoelektrischen Elementen (3) zur Auslenkung der Schneidspitze (4) versehen ist,
   - einen Regler (21), dessen Eingang mit dem Handstück (2) elektrisch verbunden ist, um einen Istwert zu empfangen, der proportional zu der an den piezoelektrischen Elementen (3) induzierten Spannung des stets in der Eigenfrequenz schwingenden Handstückes (2) ist, wobei der Regler (21) geeignet ist, den Istwert mit einem vorgegebenen Sollwert (22) zu vergleichen und somit eine Stellgröße (23) zu bestimmen,
   - einen Leistungsverstärker (24), dessen Eingang mit dem Regler (21) verbunden ist, um die Stellgröße (23) zu empfangen, und dessen Ausgang mit dem Handstück (2) verbunden ist, wobei gleichzeitig mit dem Empfangen des Istwertes das Handstück (2) ansteuerbar ist und eine Leistungsabgabe an das Handstück (2) erfolgen kann, so dass eine unmittelbare Rückkopplung erreichbar ist und somit das Handstück (2) stets in seiner Eigenfrequenz mit einer der Stellgröße (23) entsprechenden Auslenkung der Schneidspitze (4) schwingen kann.

2. Phakoemulsifikationsvorrichtung (1) nach Anspruch 1, wobei zwischen dem Regler (21) und dem Handstück (2) ein Übertrager (25) geschaltet ist, welcher an seinem Eingang mit dem Handstück (2) und an seinem Ausgang mit dem Regler (21) verbunden ist.

3. Phakoemulsifikationsvorrichtung (1) nach Anspruch 1 oder 2, wobei sich die piezoelektrischen Elemente (3) durch einen elektrischen Impuls so anregen lassen, dass eine größere Schwingungsamplitude der Schneidspitze (4) im Vergleich zum weißen Rauschen erreicht wird.

4. Phakoemulsifikationsvorrichtung (1) nach einem der vorhergehenden Ansprüche, wobei die vom Verstärker (21) zugeführte Energie wiederholt vollständig unterbrochen werden kann, so dass jeweils nach einer Schwingphase eine Ruhephase folgt.

5. Phakoemulsifikationsvorrichtung (1) nach Anspruch 4, wobei die Vorrichtung so betreibbar ist, dass je Sekunde eine Anzahl von Ruhephasen erzielbar ist, welche mindestens 1 ist und höchstens der Betrag von 1 Prozent der Eigenfrequenz des Handstückes ist.

6. Phakoemulsifikationsvorrichtung (1) nach Anspruch 4 oder 5, wobei die dem Handstück (4) zugeführte Energie von einer Schwingphase zur darauf folgenden Schwingphase in ihrer Höhe variierbar ist.

7.  Phakoemulsifikationsvorrichtung (1) nach einem der Ansprüche 1 bis 3, wobei die vom Verstärker (24) zugeführte Energie so geregelt werden kann, dass nach einer ersten Schwingphase, in der die Schneidspitze mit einer Amplitude betrieben wird, mit der die Linse (7) emulsifiziert werden kann, eine zweite Schwingphase folgt, in der die Schneidspitze mit einer Amplitude betrieben wird, mit der die Linse (7) nicht emulsifiziert werden kann.

8.  Phakoemulsifikationsvorrichtung (1) nach einem der vorherigen Ansprüche, wobei das Handstück (2) mit der Schneidspitze eine Eigenfrequenz von 20 bis 100 kHz, vorzugsweise von 40 kHz aufweist.

9.  Phakoemulsifikationsvorrichtung (1) nach einem der vorherigen Ansprüche, wobei die Schneidspitze (4) so betreibbar ist, dass sie eine mechanische Leistung zwischen 0 und 3,4 Watt abgibt.

10. Phakoemulsifikationssystem mit einer Phakoemulsifikationsvorrichtung (1) nach einem der Ansprüche 1 bis 9, einer Irrigationsvorrichtung, einer Aspirationsvorrichtung und einer Steuerungsvorrichtung zum Betreiben der Phakoemulsifikationsvorrichtung, der Irrigationsvorrichtung und der Aspirationsvorrichtung.

**Claims**

1.  Phacoemulsification apparatus (1), having:

    - a handpiece (2) which has a cutting tip (4) for emulsifying a lens (7), wherein the handpiece (2) is provided with piezoelectric elements (3) for deflecting the cutting tip (4);
    - a regulator (21), the input of which is electrically connected to the handpiece (2) in order to receive an actual value which is proportional to the voltage, induced on the piezoelectric elements (3), of the handpiece (2) constantly oscillating at the natural frequency, wherein the regulator (21) is suitable for comparing the actual value with a prescribed target value (22) and hence determining a controlled variable (23);
    - a power amplifier (24), the input of which is connected to the regulator (21) in order to receive the controlled variable (23), and the output of which is connected to the handpiece (2), wherein at the same time as the actual value is received the handpiece (2) can be actuated and power can be output to the handpiece (2), so that direct feedback can be achieved and hence the handpiece (2) can constantly oscillate at the natural frequency thereof with deflection of the cutting tip (4) in line with the controlled variable (23).

2.  Phacoemulsification apparatus (1) according to Claim 1, wherein the regulator (21) and the handpiece (2) have a transformer (25) connected between them, the input of which is connected to the handpiece (2) and the output of which is connected to the regulator (21).

3.  Phacoemulsification apparatus according to Claim 1 or 2, wherein the piezoelectric elements (3) can be excited by an electrical pulse such that a larger oscillation amplitude for the cutting tip (4) is achieved in comparison with white noise.

4.  Phacoemulsification apparatus (1) according to one of the preceding claims, wherein the energy supplied by the amplifier (21) can be repeatedly interrupted completely, so that a respective oscillation phase is followed by a quiescent phase.

5.  Phacoemulsification apparatus (1) according to Claim 4, wherein the apparatus can be operated such that every second a number of quiescent phases can be attained which is at least 1 and is no more than the absolute value of 1 percent of the natural frequency of the handpiece.

6.  Phacoemulsification apparatus (1) according to Claim 4 or 5, wherein the energy supplied to the handpiece (4) can be varied in level from one oscillation phase to the next oscillation phase.

7.  Phacoemulsification apparatus (1) according to one of Claims 1 to 3, wherein the energy supplied by the amplifier (24) can be regulated such that a first oscillation phase, in which the cutting tip is operated at an amplitude which can be used to emulsify the lens (7), is followed by a second oscillation phase, in which the cutting tip is operated at an amplitude which cannot be used to emulsify the lens (7).

8.  Phacoemulsification apparatus (1) according to one of the preceding claims, wherein the handpiece (2) with the cutting tip has a natural frequency of between 20 and 100 kHz, preferably of 40 kHz.

**9.** Phacoemulsification apparatus (1) according to one of the preceding claims, wherein the cutting tip (4) can be operated such that it outputs a mechanical power between 0 and 3.4 watts.

**10.** Phacoemulsification system having a phacoemulsification apparatus (1) according to one of Claims 1 to 9, an irrigation apparatus, an aspiration apparatus and a control apparatus for operating the phacoemulsification apparatus, the irrigation apparatus and the aspiration apparatus.

**Revendications**

**1.** Dispositif de phacoémulsion (1) comprenant:

- une pièce à main (2), laquelle comporte une pointe coupante (4) servant à l'émulsion d'une lentille (7), la pièce à main (2) étant pourvue d'éléments piézoélectriques (3) servant à infléchir la pointe coupante (4),
- un régulateur (21) dont l'entrée est électriquement reliée à la pièce à main (2) de façon à recevoir une valeur réelle qui est proportionnelle à la tension induite aux éléments piézoélectriques (3) de la pièce à main (2) maintenue en oscillation permanente à la fréquence de résonance, le régulateur (21) étant conçu de façon à comparer à une valeur de consigne prescrite (22) la valeur réelle et à déterminer ainsi une grandeur de commande (23),
- un amplificateur de puissance (24) dont l'entrée est reliée au régulateur (21) de façon à recevoir la grandeur de commande (23) et dont la sortie est reliée à la pièce à main (2) et la pièce à main (2) pouvant être commandée simultanément avec la réception de la valeur réelle et une sortie de puissance pouvant avoir lieu à la pièce à main (2) de manière à pouvoir obtenir une rétroaction directe et que de ce fait la pièce à main (2) puisse vibrer en continu à sa fréquence de résonance avec une inflexion de la pointe coupante (4) correspondant à la grandeur de commande (23).

**2.** Dispositif de phacoémulsion (1) selon la revendication 1, un transmetteur (25) étant connecté entre le régulateur (21) et la pièce à main (2), lequel est relié à son entrée à la pièce à main (2) et à sa sortie au régulateur (21).

**3.** Dispositif de phacoémulsion (1) selon la revendication 1 ou 2, les éléments piézoélectriques (3) pouvant être excités par une impulsion électrique de manière à pouvoir obtenir une amplitude d'oscillation de la pointe coupante (4) plus importante comparée au bruit blanc.

**4.** Dispositif de phacoémulsion (1) selon l'une quelconque des revendications précédentes, l'énergie fournie par l'amplificateur (21) pouvant être interrompue complètement de manière répétée de manière qu'à chaque fois une phase de repos a lieu après une phase d'oscillation.

**5.** Dispositif de phacoémulsion (1) selon la revendication 4, le dispositif pouvant être mis en oeuvre de manière à ce qu'à chaque seconde un nombre de phases de repos peut être obtenu, lequel est au moins de 1 et est au maximum le montant de 1 pour cent de la fréquence de résonance de la pièce à main.

**6.** Dispositif de phacoémulsion (1) selon la revendication 4 ou 5, l'énergie fournie à la pièce à main (4) pouvant être modifiée dans son importance d'une phase d'oscillation à la phase d'oscillation qui s'ensuit.

**7.** Dispositif de phacoémulsion (1) selon l'une quelconque des revendications 1 à 3, l'énergie fournie par l'amplificateur (24) pouvant être réglée de manière qu'après une première phase d'oscillation dans laquelle la pointe coupante est mise en oeuvre avec une amplitude permettant d'émulsifier la lentille (7), une deuxième phase d'oscillation a lieu dans laquelle la pointe coupante est mise en oeuvre à une amplitude à laquelle la lentille (7) ne peut pas être émulsifiée.

**8.** Dispositif de phacoémulsion (1) selon l'une quelconque des revendications précédentes, la pièce à main (2) avec la pointe coupante présentant une fréquence de résonance de 20 à 100 kHz, de préférence de 40 kHz.

**9.** Dispositif de phacoémulsion (1) selon l'une quelconque des revendications précédentes, la pointe coupante (4) pouvant être mise en oeuvre de manière qu'elle fournisse une puissance mécanique se situant entre 0 et 3,4 Watt.

**10.** Système de phacoémulsification avec un dispositif de phacoémulsion (1) selon l'une quelconque des revendications 1 à 9, un dispositif d'irrigation, un dispositif d'aspiration et un dispositif de commande pour mettre en oeuvre le dispositif de phacoémulsification, le dispositif d'irrigation et le dispositif d'aspiration.

Fig. 1

Stand der Technik

Fig. 2

EP 2 187 851 B1

Fig. 3

EP 2 187 851 B1

Fig. 4

Fig. 5

EP 2 187 851 B1

Fig. 6

EP 2 187 851 B1

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6997935 B2

- US 6203516 B